# EUROPÄISCHE PATENTSCHRIFT

(11) **EP 0 601 318 B1**
(45) Veröffentlichungstag und Bekanntmachung des Hinweises auf die Patenterteilung: **24.04.2002**
(21) Anmeldenummer: 93117202.7
(22) Anmeldetag: 23.10.1993
(51) Int. Cl.: G01N 33/535, G01N 33/532

(54) **Verfahren zur Herstellung von Konjugaten aus einem spezifischen Bindungspartner und einem kohlenhydrathaltigen Protein**
Process for production of a conjugate from a specific binding partner and a carbohydrate containing protein
Procédé de préparation de conjugués d'un membre spécifique d'une réaction et d'une proteine glycosylée

(30) Priorität: 06.11.1992 DE 4237479
(43) Veröffentlichungstag der Anmeldung: 15.06.1994
(73) Patentinhaber: Dade Behring Marburg GmbH, 35001 Marburg (DE)
(72) Erfinder: Pollner, Reinhold, D-35041 Marburg (DE); Noah, Michael, D-35041 Marburg (DE); Nau, Günther, D-35043 Marburg (DE)

(56) Entgegenhaltungen:
- EP-A- 0 209 155
- US-A- 4 002 532
- US-A- 4 587 044
- THE JOURNAL OF HISTOCHEMISTRY AND CYTOCHEMISTRY, Bd.22, Nr.12, Dezember 1974, NEW YORK Seiten 1084 - 1091 NAKANE ET AL. 'Peroxidase-labeled antibody. A new method of conjugation'

## Beschreibung

Die Erfindung betrifft ein Verfahren zur Herstellung von Konjugaten aus einem spezifischen Bindungspartner und einem kohlenhydrathaltigen Protein, durch dieses Verfahren herstellbare Konjugate und deren Verwendung in Enzymimmunoassays.

Nachdem Yalow und Berson (R. S. Yalow, S. A. Berson (1960) J. Clin. Invest. 39, 1157 - 1175) den ersten Radioimmunoassay und Engvall und Perlmann (E. Engvall, P. Perlmann (1971) Immunochemistry 8, 871 - 874) sowie van Weemen und Schuurs (B. K. van Weemen, A. H. W. M. Schuurs (1971) FEBS-Letters 15, 232 - 236) die ersten Enzymimmunoassays (EIA) beschrieben haben, wurden diese Techniken weltweit zur Bestimmung von verschiedensten Analyten eingesetzt.

Immunologische Nachweismethoden zeichnen sich im allgemeinen durch außerordentliche Spezifität und Sensitivität aus.

Diese Techniken finden heute nicht nur in Forschungslaboratorien ihre Anwendung, sondern haben bereits seit langem Einzug in die Routine-Diagnostik und Analytik gefunden.

Bei einem Enzymimmunoassay zur Bestimmung von niedrig konzentrierten Analyten kommen immunologische Bindungspartner, wie z. B. Antigene, Haptene, Antikörper oder Derivate oder Fragmente von Antikörpern zum Einsatz, die mit einem Markierungsenzym über eine kovalente Bindung gekoppelt werden. Die erhaltenen Kopplungsprodukte zwischen dem immunologischen Bindungspartner und dem Markierungsenzym werden allgemein Konjugate genannt.

Als Markierungsenzyme für Enzymimmunoassays werden häufig alkalische Phosphatase, β-Galaktosidase und Meerrettich-Peroxidase verwendet, wobei als Substrate chromogene, fluorogene oder lumineszente Verbindungen ihre Verwendung finden. Darüber hinaus sind andere Markierungen, wie z. B. mit Fluoreszenzfarbstoffen oder chemolumineszenzfähigen Molekülen dem Fachmann bekannt.

Als Festphase werden konkave Formkörper, wie z. B. Röhrchen oder Vertiefungen in Form von Mikrotitrationsplatten und konvexe Formkörper, wie z. B. Kugeln, Mikropartikel (Latex-Partikel), Stäbchen und Magnetpartikel, verwendet. Außerdem kommen planare Festphasen, wie z. B. Teststreifen, zur Anwendung. Als Materialien für die Festphase von Enzymimmunoassays werden häufig Polystyrol oder auch andere Materialien verwendet.

Die möglichen Testprinzipien für einen Enzymimmunoassay, die Zusammensetzung von Proben-, Inkubations-, Waschpuffern sowie die Substrat-/Chromogen-Reagenzien sind dem Fachmann bekannt.

Die Kopplung des Markierungsenzyms mit dem immunologischen Bindungspartner kann z. B. mit der Glutaraldehyd-Methode (Ein- oder Zwei-Stufenmethode, S. Avrameas (1969) Immunochemistry 6, 43 - 52), der Perjodat-Methode (P. K. Nakane und A. Kawaoi (1974) J. Histochem. Cytochem. 22, 1084 - 1091) oder mit Hilfe von heterobifunktionellen Konjugationsmethoden (F. Wold (1972) Methods Enzymol. 25, 623 - 651) erfolgen (Übersichtsartikel: E. Ishikawa et al. (1983) J. Immunoassay 4, 209 - 327). Der Einsatz von heterobifunktionellen Reagenzien, die als Brückenbildner die Quervernetzung des Markierungsenzyms und des immunologischen Bindungspartners ermöglichen, erlaubte die Herstellung von wesentlich besser charakterisierten Konjugaten. Dabei erwies es sich als besonders vorteilhaft, daß bei der heterobifunktionellen Methode die Bindungsaffinität der immunologischen Komponente weniger beeinträchtigt wurde als bei der Glutaraldehyd- und der Perjodat-Methode (E. Ishikawa et al. (1984) J. Immunoassay 4, 209 - 327).

Im US-Patent Nr. 4002532 wurde die Verwendung von u.a. Diaminen zur Verbesserung der Spezifität von Immuntesten vorgeschlagen. Die Wirkung des Diamins besteht hierbei hauptsächlich in der Stabilisierung des Konjugats und der Verhinderung einer Präzipitation.

Für die Diagnostik z. B. von Hepatitis B Virus Infektionen werden Radio-und Enzymimmunoassays in zunehmendem Maße eingesetzt, wobei in den letzten Jahren sowohl die Sensitivität als auch die Spezifität des Nachweises einer Hepatitis B Virus Infektion entscheidend verbessert werden konnte. Die viralen Antigene und die dazu korrespondierenden Antikörper erscheinen nach einer Infektion mit dem Hepatitis B Virus im Serum des Betroffenen in einer bestimmten Reihenfolge, die im Zusammenhang mit der viralen Replikation und der Reaktion des Immunsystems des Wirts steht. Von den verschiedenen diagnostischen und serologischen Markern besitzt die Bestimmung des Hepatitis B Oberflächen Antigens (HBsAg) sowohl für die Vermeidung der Übertragung von infektiösem Blut oder Plasma in der Transfusionsmedizin als auch in der Differentialdiagnose eine überragende Bedeutung. Da man davon ausgehen muß, daß auch kleinste Virusmengen infektiös sind, werden höchste Anforderungen an die Nachweisgrenze eines Tests für HBsAg gestellt.

Enzymimmunoassays können nach verschiedenen, dem Fachmann bekannten Prinzipien aufgebaut sein (z. B. Sandwich-Prinzip, kompetitiv, indirekt usw.). Beim HBsAg-Nachweis kommt im allgemeinen das Sandwich-Prinzip zur Anwendung, wobei eine mit Anti-HBs-Antikörpem beschichtete Festphase mit der zu untersuchenden Probe inkubiert wird. Falls HBsAg in der untersuchten Probe vorhanden ist, erfolgt seine Bindung an die immobilisierten Antikörper. Mittels eines zweiten Antikörpers, der mit einem Markierungsenzym gekoppelt ist, bildet sich ein ternärer Komplex (Sandwich). Die ungebundenen Reaktanden werden danach mit mehreren Waschschritten abgetrennt.

Nach Zugabe eines Substrat-/Chromogengemisches wird die Bildung des entstandenen Sandwich-Komplexes durch eine photometrisch zu detektierende Farbreaktion nachgewiesen. Enzymimmunoassays für HBsAg nach dem oben beschriebenen Prinzip sind kommerziell erhältlich und auch in der Fachliteratur eingehend beschrieben.

Die in der Fachliteratur beschriebenen und die kommerziell erhältlichen Enzymimmunoassays für den Nachweis von z. B. HBsAg zeichnen sich durch eine hohe Sensitivität und Spezifität aus. Trotz der guten Spezifität der HBsAg-Nachweisverfahren sind Seren bekannt, deren Spender nachweislich nicht Hepatitis B positiv sind, die aber trotzdem falsch-positive Resultate liefern ("Problemseren"). Die Unterscheidung zwischen falsch-positiven und positiven Proben kann generell durch die Wiederholung des HBsAg-Nachweistests bzw. mit Hilfe diverser Bestätigungstests getroffen werden. Dies ist für den Anwender allerdings mit einem zusätzlichen Aufwand an Zeit und Kosten verbunden. Es ist daher anzustreben, die Gefahr von falsch-positiven Ergebnissen bereits beim eigentlichen Initial-Test möglichst gering zu halten.

Aus der EP 0 209 155 ist ein Enzymimmunoassay für die Bestimmung von Thyroxin bekannt, bei dem eine Perjodatoxidation des Markierungsenzyms zu Derivaten führt, die auch mit "Problemseren" richtige Ergebnisse liefern. Aufgrund des kompetitiven Prinzips werden dabei falsch-erniedrigte Extinktionswerte und damit falsch-erhöhte Analytwerte gemessen. Eine Verbesserung der Spezifität wurde dadurch erzielt, daß man das Markierungsenzym, welches einen Kohlenhydratanteil enthält, vor oder nach Konjugation mit dem immunologischen Liganden mit Perjodsäure oder einem Alkalisalz derselben in wäßrigem Medium behandelt und das erhaltene Oxidationsprodukt mit Natriumborhydrid reduziert. Die eigentliche Kopplungsreaktion erfolgt dabei unter Anwendung einer an den Proteinanteilen des Markierungsenzyms und immunologischen Liganden angreifenden heterobifunktionellen Konjugationsmethode.

Aufgabe der vorliegenden Erfindung war es nun, diesen Nachteil in der Spezifität zu beseitigen und mit Hilfe einer verbesserten Technik Konjugate zu erzeugen, die auch bei problematischen Seren richtige Ergebnisse liefern.

Überraschenderweise stellte sich heraus, daß mit Hilfe der erfindungsgemäßen Konjugationstechnik nicht nur Nachteile in der Spezifität eliminiert werden konnten, sondern auch eine Verbesserung der Sensitivität des HBsAg-Nachweises erzielt wurde.

Die Erfindung betrifft daher ein Verfahren zur Herstellung eines Konjugates aus einem spezifischen Bindungspartner und einem kohlenhydrathaltigen Protein, das die folgenden Schritte einschließt:
a) Oxidation des kohlenhydrathaltigen Proteins mit Perjodsäure oder einem entsprechenden Alkalisalz in gepufferter Lösung,
b) Einführung von zusätzlichen Aminogruppen in das oxidierte Protein durch Umsetzung mit einem Diamin und reduktiver Stabilisierung und anschließender
c) Kopplung des spezifischen Bindungspartners und des aktivierten Proteins über ein heterobifunktionelles Reagenz.

Bevorzugt ist dabei ein Verfahren nach Anspruch 1, wobei der Kohlenhydratanteil des Proteins 4 bis 80 % Gew. beträgt.

Bevorzugterweise erfolgt dabei die Oxidation bei einem pH von 4 bis 8.

Die Umsetzung mit dem Diamin und reduktive Stabilisierung erfolgt bevorzugterweise bei einem pH von 7 bis 9,5.

Ganz bevorzugterweise ist das kohlenhydratreiche Protein ein Enzym, besonders bevorzugterweise Meerrettich-Peroxidase.

Bevorzugt ist auch ein solches Verfahren, bei welchem es sich bei der Umsetzung in Schritt b) um ein Diamin der Formel H₂N-(X)ₙ-NH₂ handelt,wobei bevorzugterweise X eine aliphatische Verbindung der Formel -CH₂- oder -(CH₂)ₙ-O-(CH₂)ₙ- ist und n eine Zahl zwischen 2 bis 12 ist, ganz bevorzugterweise wird als Diamin Diaminohexan verwendet.

Gegenstand der Erfindung ist auch ein Konjugat, herstellbar nach dem erfindungsgemäßen Verfahren.

Gegenstand der Erfindung ist ferner die Verwendung eines solchen Konjugates in immunologischen Nachweisverfahren, insbesondere in einem Enzymimmunoassay, bevorzugterweise in einem Einschritt-Enzymimmunoassay.

Die erfindungsgemäße Konjugationstechnik zeichnet sich dadurch aus, daß die eigentliche Kopplung zwischen dem Markierungsenzym und dem immunologisch-wirksamen Liganden nicht über den Proteinanteil des Markierungsenzyms, sondern über die oxidierten Kohlenhydratanteile bzw. nachfolgend eingeführten Aminogruppen des Markierungsenzyms erfolgt.

Um die Spezifität und Sensitivität für den Nachweis von HBsAg zu verbessern, wurde ein Verfahren zur Herstellung von Konjugaten gefunden, das zum einen als Kopplungskomponente ein kohlenhydrathaltiges Markierungsenzym, wie z. B. Meerrettich-Peroxidase (POD), Glucoseoxidase (GOD), alkalische Phosphatase (AP) und Fructosidase (Invertase) und zum anderen immunologisch aktive Bindungspartner, wie z. B. polyklonale und monoklonale Antikörper sowie deren Derivate und Fragmente, aber auch Antigene und Haptene beinhaltet.

Bei der erfindungsgemäßen Konjugationstechnik werden zunächst die Aminogruppen des Markierungsenzyms mit 1-Fluor-2,4-dinitrobenzol blockiert und danach die kohlenhydrathaltigen Seitenketten des Markierungsenzyms mit Natriumperjodat oxydiert. Die Aminogruppen müssen dabei nicht unbedingt blockiert werden, bzw. es können auch andere Substanzen als 1-Fluor-2,4-dinitrobenzol verwendet werden. Die bei der Perjodat-Oxydation erzeugten Aldehydgruppen reagieren mit den Aminogruppen eines zugefügten Diamins der Formel H₂N-(X)ₙ-NH₂ unter Ausbildung von Schiff'schen Basen, die durch Behandlung mit Natriumborhydrid reduktiv stabilisiert werden.

Die so eingeführten Aminogruppen werden mit Iminothiolan umgesetzt, wobei die dabei erzeugten freien Sulfhydrylgruppen dann über ein brückenbildendes heterobifunktionelles Reagenz (wie z. B. N-maleimidobutyryloxysuccinimid, GMBS) mit den freien Aminogruppen des jeweiligen immunologischen Bindungspartners reagieren.

Es ist dem Fachmann an sich bekannt, daß die Temperaturen bei den Umsetzungen auf die bekannten Bedingungen für die Aufrechterhaltung der Enzymaktivität des kohlenhydrathaltigen Markierungsenzyms, wie z. B. Meerrettich-Peroxidase, abzustimmen ist.

Die Oxidation erfolgt dabei vorzugsweise bei pH-Werten zwischen 4,0 und 8,0 - bevorzugterweise bei pH 7,0 in gepufferter Lösung. Die reduktive Stabilisierung der gebildeten Schiff'schen Basen wird vorzugsweise bei pH-Werten zwischen 7,0 und 9,5 - bevorzugterweise bei pH 8,5 durchgeführt.

Aufgrund der guten Temperaturstabilität der POD können die oben genannten Reaktionen vorzugsweise bei Temperaturen zwischen 0 °C und +37 °C, bevorzugterweise aber bei Raumtemperatur, erfolgen.

Das folgende Beispiel soll die Erfindung erläutern, sie aber in keiner Weise einschränken. Dies gilt insbesondere für andere kohlenhydrathaltigen Markierungsenzyme außer der POD, wie z. B. GOD, AP und Invertase, die im Rahmen der Erfindung als geeignet erscheinen.

### Beispiel

### a) Herstellung von Konjugat I

Der Antikörper wird mit einem heterobifunktionellen Reagenz umgesetzt (Tanimori et al. (1983) J. Imm. Meth. 62, 123 - 131), danach mit SH-aktivierter Peroxidase (King et al. (1978) Biochemistry 17, 1499 - 1506) inkubiert und anschließend gelchromatographisch aufgereinigt.

### b) Herstellung von Konjugat II

Das Markierungsenzym Peroxidase wird mit 1-Fluor-2,4-dinitrobenzol umgesetzt und danach mit Perjodat oxidiert. Die so erhaltenen Aldehydgruppen werden mit den Aminogruppen des Antikörpers gekoppelt. Die entstandenen Schiffschen Basen werden dann mit Natriumborhydrid reduziert (P. Tijssen (1985) Laboratory Techniques in Biochemistry and Molecular Biology, 223 - 241). Die Reaktionsprodukte werden anschließend von der freien Peroxidase und dem freien Antikörper gelchromatographisch aufgereinigt.

### c) Herstellung von Konjugat III

20 mg Peroxidase werden in 20 mM Natriumphosphat-Puffer (pH 7,0) gelöst und mit 0,4 ml 1-Fluor-2,4-dinitrobenzol-Lösung (10 µl 1-Fluor-2,4-dinitrobenzol + 1,93 ml Ethanol) versetzt. Der Ansatz wird 1 h bei Raumtemperatur inkubiert, dann 0,6 ml einer 0,25 M Natriumperjodat-Lösung zugegeben und weitere 30 min bei Raumtemperatur unter Lichtausschluß inkubiert.

Der Reagenzüberschuß wird anschließend durch Gelchromatographie entfernt, wobei die Säule vorher mit 20 mM Natriumphosphat-Puffer (pH 7,0) äquilibriert worden war. Die Konzentration der so voraktivierten Peroxidase wird bei 403 nm bestimmt (ε = 2,275 g⁻¹.L.cm⁻¹).

2,5 ml Peroxidase (7,5 mg) werden mit 7,0 ml 0,2 M Natriumhydrogencarbonat/150 mM NaCl (pH 8,5) verdünnt, danach mit 1,0 ml Diaminohexan-Lösung (5,8 mg/ml in 0,2 M Natriumcarbonat/150 mM NaCl pH 8,5) versetzt und 2 h bei Raumtemperatur unter Lichtausschluß inkubiert. Anschließend wird diese Lösung mit 0,9 ml einer Natriumborhydrid-Lösung (5 mg/ml) versetzt und 2 h im offenen Gefäß stehen gelassen. Der Reaktionsansatz wird mit 1,2 ml einer 1 M Ethanolamin/HCl-Lösung (pH 8,0) versetzt und über Nacht bei +4 °C inkubiert. Am nächsten Tag wird die Peroxidase-Lösung ankonzentriert und in 25 mM Natriumtetraborat umgepuffert. Die Peroxidasekonzentration sollte 1,5 - 2,0 mg/ml betragen. 8 mg Peroxidase-Lösung werden mit 83 µl einer 1 M Iminothiolan-Lösung (gelöst in Methanol) versetzt und 2 h bei Raumtemperatur inkubiert, anschließend der Reagenzüberschuß mit 0,1 M Natriumphosphat-Puffer (pH 6,0) gelchromatographisch entfernt und die Konzentration bei 403 nm bestimmt. 4 mg Antikörper (2 mg/ml Lösung in 0,1 M Lithiumborat-Puffer pH 8,0) werden mit 70 µl GMBS-Lösung (3 mg/ml in Dioxan) vermischt, 1 h bei Raumtemperatur inkubiert und anschließend in 0,1 M Natriumphosphat-Puffer (pH 6,0) umgepuffert.

Zu 2,7 ml aktivierten Antikörper (3,25 mg) werden 1,2 ml aktivierte Peroxidase-Lösung (4,3 mg) gegeben, 2 h bei Raumtemperatur inkubiert und danach mit 0,5 ml einer 0,1 M N-Ethylmaleimid-Lösung abgestoppt.

### d) HBsAg-Enzymimmunoassay

Ein typischer Enzymimmunoassay, wie z. B. Enzygnost ®HBsAg monoclonal II, für den Nachweis von HBsAg basiert auf dem Einschritt-Sandwich-Prinzip. Das in der untersuchten Probe (100 µl) enthaltene HBsAg reagiert simultan mit dem in der Vertiefung der Mikrotitrationsplatte fixierten polyklonalen anti-HBs-Antikörper und dem monoklonalen Peroxidase-konjugierten anti-HBs-Antikörper (anti-HBs/POD-Konjugat). Die Inkubationszeit beträgt 90 min bei +37 °C. Nach Entfernen der ungebundenen Reaktanden durch Absaugen und viermaliges Waschen wird die Menge des ge-bundenen Konjugates durch Zugabe von 100 µl Substrat/ Chromogen-Lösung bestimmt (30 min, Raumtemperatur, lichtgeschützt). Die enzymatische Umsetzung des Chromogens Tetramethylbenzidin-dihydrochlorid wird durch Zusatz von 100 µl 0,5 N Schwefelsäure unterbrochen und die Extinktion bei 450 nm photometrisch bestimmt. Die gemessene Extinktion ist der in der Probe vorhandenen HBsAg-Konzentration proportional.

Im Enzymimmunoassay konnte gezeigt werden, daß mit den erfindungsgemäßen Konjugaten zum einen eine entscheidende Verbesserung der Sensitivität für den Nachweis von HBsAg möglich ist, aber auch falsch-positive Signale bei Problemseren nicht mehr auftraten. Tabelle I zeigt die Ergebnisse einer HBsAg-Enzymimmunobestimmung mit Konjugaten, die
a) über ein heterobifunktionelles Reagenz (= Konjugat I)
b) nach der herkömmlichen Nakane-Methode (= Konjugat II)
c) nach der erfindungsgemäßen Methode (= Konjugat III)
erhalten wurden. Verglichen werden negative Kontrolle und HBsAg-Proben, die an einem Standardmaterial des Paul-Ehrlich-Instituts (Frankfurt, BRD) kalibriert wurden.

Die nach linearer Regression und unter Berücksichtigung eines threshold-Wertes von 50 mE berechnete Sensitivität ist ebenfalls aufgeführt.

**Tabelle I**

| **Sensitivitätsvergleich der Konjugate I-III** | | | |
|---|---|---|---|
| **Probe** | **Konjugat I** | **Konjugat II** | **Konjugat III** |
| neg. Kontr. | 37 mE | 64 mE | 44 mE |
| 0,05 U/ml | 69 mE | 56 mE | 93 mE |
| 0,10 U/ml | 82 mE | 74 mE | 153 mE |
| 0,20 U/ml | 133 mE | 114 mE | 276 mE |
| 0,50 U/ml | 301 mE | 241 mE | 639 mE |
| 1,00 U/ml | 584 mE | 525 mE | 1247 mE |
| 2,00 U/ml | 1257 mE | 1051 mE | 2517 mE |
| **Sensitivität** | **0,12 U/ml** | **0,20 U/ml** | **0,06 U/ml** |

Tabelle II zeigt die Ergebnisse einer HBsAg-Enzymimmunobestimmung mit Konjugaten, die
a) über ein heterobifunktionelles Reagenz (= Konjugat I)
b) nach der herkömmlichen Nakane-Methode (= Konjugat II)
c) nach der erfindungsgemäßen Methode (= Konjugat III)
erhalten wurden. Verglichen werden negative Kontrolle, cut-off-Wert, drei Normalseren (NS 1-NS 3) und zehn "Problemseren" (PS 1-PS 10). Der threshold-Wert wurde mit 50 mE angesetzt, wobei sich der cut-off-Wert aus der Addition von threshold-Wert und negativer Kontrolle berechnet.

**Tabelle II:**

| **Spezifitätsvergleich der Konjugate I-III** | | | |
|---|---|---|---|
| **Probe** | **Konjugat I** | **Konjugat II** | **Konjugat III** |
| neg. Kontr. | 16 mE | 52 mE | 60 mE |
| cut-off | 66 mE | 102 mE | 110 mE |
| NS 1 | 20 mE | 73 mE | 55 mE |
| NS2 | 25 mE | 77 mE | 60 mE |
| NS3 | 28 mE | 80 mE | 66 mE |
| PS 1 | 105 mE | 73 mE | 74 mE |
| PS 2 | 248 mE | 41 mE | 39 mE |
| PS 3 | 455 mE | 27mE | 42 mE |
| PS 4 | 364 mE | 87 mE | 68 mE |
| PS 5 | 251 mE | 90 mE | 56 mE |
| PS 6 | 569 mE | 31 mE | 27 mE |
| PS 7 | 3283 mE | 76 mE | 56 mE |
| PS 8 | 14 mE | 106 mE | 85 mE |
| PS 9 | 9 mE | 105 mE | 78 mE |
| PS 10 | 29 mE | 139 mE | 81 mE |

## Patentansprüche

1. Verfahren zur Herstellung eines Konjugates aus einem spezifischen Bindungspartner und einem kohlenhydrathaltigen Protein, das die folgenden Schritte einschließt:
a) Oxidation des kohlenhydrathaltigen Proteins mit Perjodsäure oder einem entsprechenden Alkalisalz in gepufferter Lösung,
b) Einführung von zusätzlichen Aminogruppen in das oxidierte Protein durch Umsetzung mit einem Diamin und reduktiver Stabilisierung und anschließender
c) Kopplung des spezifischen Bindungspartners und des aktivierten Proteins über ein heterobifunktionelles Reagenz.

2. Verfahren nach Anspruch 1, wobei der Kohlenhydratanteil des Proteins 4 bis 80% Gew. beträgt.

3. Verfahren nach Anspruch 1, wobei die Oxidation bei einem pH von 4-8 erfolgt.

4. Verfahren nach Anspruch 1, wobei die Umsetzung mit dem Diamin und reduktive Stabilisierung bei einem pH von 7 bis 9,5 erfolgt.

5. Verfahren nach Anspruch 1 **dadurch gekennzeichnet, daß** das kohlenhydratreiche Protein ein Enzym ist.

6. Verfahren nach Anspruch 5 **dadurch gekennzeichnet, daß** das Enzym Meerrettich-Peroxidase ist.

7. Verfahren nach Anspruch 1 **dadurch gekennzeichnet, daß** es sich bei der Umsetzung in Schritt b) um ein Diamin der Formel H₂N-(X)ₙ-NH₂ handelt.

8. Verfahren nach Anspruch 7 **dadurch gekennzeichnet, daß** X eine aliphatische Verbindung der Formel ―(CH₂)- oder ―(CH₂)ₙ-O-(CH₂)ₙ- ist, wobei n bevorzugterweise eine Zahl zwischen 2 bis 12 ist.

9. Verfahren nach Anspruch 7 **dadurch gekennzeichnet, daß** als Diamin Diaminohexan verwendet wird.

10. Konjugat, herstellbar nach dem Verfahren gemäß mindestens einem der Ansprüche 1-9.

11. Verwendung eines Konjugates nach Anspruch 10 in immunologischen Nachweisverfahren.

12. Verwendung eines Konjugates nach Anspruch 10 in einem Enzymimmunoassay.

13. Verwendung nach Anspruch 12 **dadurch gekennzeichnet, daß** es sich um einen Einschritt-Enzymimmunoassay handelt.

## Claims

1. A process for preparing a conjugate consisting of a specific binding partner and a carbohydrate-containing protein, which process includes the following steps:
a) oxidizing the carbohydrate-containing protein with periodic acid or with a corresponding alkali metal salt in buffered solution,
b) introducing additional amino groups into the oxidized protein by reaction with a diamine and reductive stabilization, and subsequently
c) coupling the specific binding partner and the activated protein via a heterobifunctional reagent.

2. The process as claimed in claim 1, wherein the carbohydrate moiety of the protein is 4 to 80 % by weight.

3. The process as claimed in claim 1, wherein the oxidation is effected at a pH of 4 to 8.

4. The process as claimed in claim 1, wherein the reaction with the diamine, and reductive stabilization, are effected at a pH of 7 to 9.5.

5. The process as claimed in claim 1, wherein the carbohydrate-rich protein is an enzyme.

6. The process as claimed in claim 5, wherein the enzyme is horseradish peroxidase.

7. The process as claimed in claim 1, wherein a diamine of the formula H₂N-(X)ₙ-NH₂ is used for the reaction in step b).

8. The process as claimed in claim 7, wherein X is an aliphatic combining unit of the formula -(CH₂)- or -(CH₂)ₙ-O-(CH₂)ₙ-, where n is preferably a number from 2 to 12.

9. The process as claimed in claim 7, wherein diaminohexane is used as the diamine.

10. A conjugate which can be prepared by the process as claimed in at least one of claims 1 - 9.

11. Use of a conjugate as claimed in claim 10 in immunological detection processes.

12. The use of a conjugate as claimed in claim 10 in an enzyme immunoassay.

13. The use as claimed in claim 12, wherein the enzyme immunoassay is a once-step enzyme immunoassay.

## Revendications

1. Procédé pour la préparation d'un conjugué d'un partenaire de liaison spécifique et d'une protéine contenant un glucide, comprenant les étapes suivantes :
a) oxydation de la protéine contenant un glucide, à l'aide d'acide periodique ou d'un sel alcalin correspondant, en solution tamponnée,
b) introduction de groupes amino supplémentaires dans la protéine oxydée, par réaction avec une diamine et stabilisation réductrice et
c) couplage subséquent du partenaire de liaison spécifique et de la protéine activée, par l'intermédiaire d'un réactif hétérobifonctionnel.

2. Procédé selon la revendication 1, dans lequel la teneur en glucide de la protéine va de 4 à 80 % en poids.

3. Procédé selon la revendication 1, dans lequel l'oxydation est effectuée à un pH de 4-8.

4. Procédé selon la revendication 1, dans lequel la réaction avec la diamine et la stabilisation réductrice s'effectuent à un pH de 7 à 9,5.

5. Procédé selon la revendication 1, **caractérisé en ce que** la protéine contenant un glucide est une enzyme.

6. Procédé selon la revendication 5, **caractérisé en ce que** l'enzyme est la peroxydase de raifort.

7. Procédé selon la revendication 1, **caractérisé en ce que** dans la réaction il s'agit dans l'étape b) d'une diamine de formule H₂N-(X)ₙ-NH₂.

8. Procédé selon la revendication 7, **caractérisé en ce que** X est un composé aliphatique de formule -(CH₂)- ou -(CH₂)ₙ-O-(CH₂)ₙ-, n étant de préférence un nombre compris entre 2 et 12.

9. Procédé selon la revendication 7, **caractérisé en ce qu'**on utilise comme diamine le diaminohexane.

10. Conjugué pouvant être préparé par le procédé selon au moins l'une des revendications 1 à 9.

11. Utilisation d'un conjugué selon la revendication 10, dans des procédés de détection immunologiques.

12. Utilisation d'un conjugué selon la revendication 10, dans un dosage immunoenzymatique.

13. Utilisation selon la revendication 12, **caractérisée en ce qu'**il s'agit d'un dosage immunoenzymatique en une seule étape.
